# FASCICULE DE BREVET EUROPEEN

(11) **EP 2 316 356 B1**
(45) Date de publication et mention de la délivrance du brevet: **23.03.2016**
(21) Numéro de dépôt: 09174374.0
(22) Date de dépôt: 28.10.2009
(51) Int. Cl.: A61B 17/16, A61B 17/14

(54) **Dispositif d'accouplement entre un arbre moteur d'un instrument chirurgical et un outil**
Kupplungsvorrichtung zwischen einer Motorwelle eines chirurgischen Instruments und einem Werkzeug
Device for coupling between a motor shaft of a surgical instrument and a tool

(43) Date de publication de la demande: 04.05.2011
(73) Titulaire: Bien-Air Holding SA, 2500 Bienne 6 (CH)
(72) Inventeur: Gigon, M. David, 2340, Le Noirmont (CH)
(74) Mandataire: Surmely, Gérard

(56) Documents cités:
- EP-A1- 1 623 677
- EP-A1- 1 974 680
- FR-A1- 2 635 676
- US-A- 5 871 493
- US-A- 5 989 257
- US-A- 6 065 966

## Description

La présente invention concerne un dispositif d'accouplement entre un arbre moteur d'un instrument chirurgical et un outil. Plus précisément, la présente invention concerne un dispositif d'accouplement entre un instrument chirurgical motorisé et un outil de rhinoplastie.

La rhinoplastie est une branche de la chirurgie esthétique qui vise à redessiner le profil du nez d'un patient. Un cas particulier est la correction par ablation osseuse de la bosse plus ou moins prononcée que l'on rencontre chez certaines personnes au niveau de l'arête du nez. Pour réaliser cette ablation, le chirurgien utilise des pièces à main motorisées qui impriment aux outils du genre scie, râpe ou rabot des mouvements spécifiques de va-et-vient. Un exemple d'une telle pièce à main est illustré à la figure 1 annexée à la présente demande de brevet. Désignée dans son ensemble par la référence numérique générale 1, la pièce à main comprend un corps 2 de forme générale cylindrique à l'intérieur duquel sont logés un moteur électrique et un arbre entraîné par ce moteur (non visibles sur le dessin). Une douille d'accouplement 4 permet de monter de manière amovible un outil 6 tel qu'une lame de scie sur l'arbre moteur. Le chirurgien tient la pièce à main 2 en main par le corps 2 de celle-ci. A cet effet, on peut prévoir une surface cannelée 8 qui va définir une zone de préhension antidérapante.

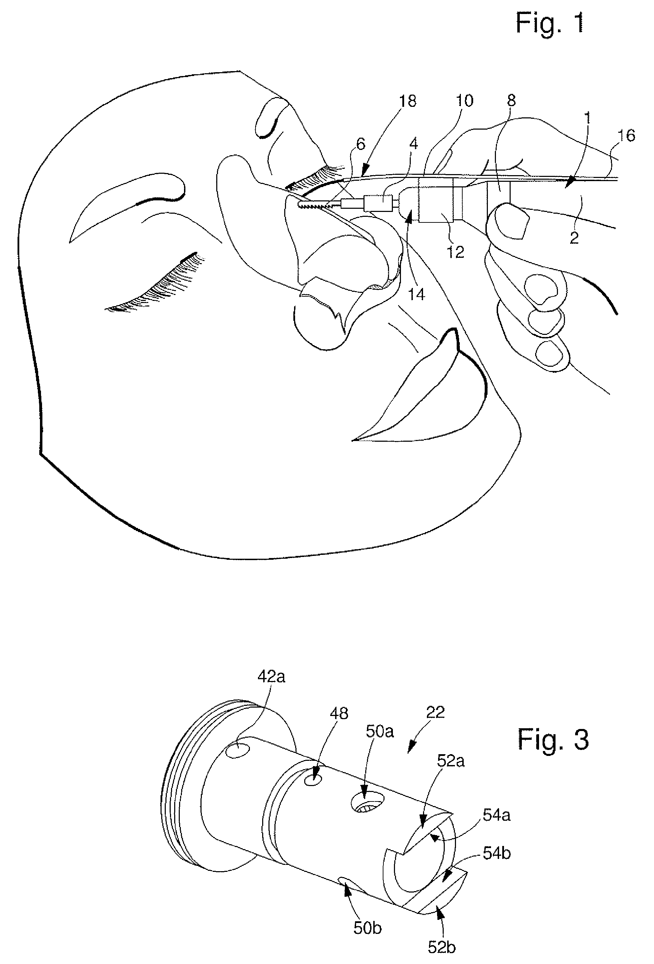

La réussite de l'opération dépend pour une part de la qualité de la pièce à main et des outils utilisés. Elle dépend pour une autre part de l'irrigation de la zone de travail en sérum physiologique. Ce sérum permet en effet de maintenir à un niveau optimum les qualités de coupe et d'abrasion des outils et d'éliminer au fur et à mesure les débris osseux et le sang. A cet effet, le corps 2 de la pièce à main 1 est pourvu, du côté de son extrémité portant l'outil 6, d'un conduit 10 d'amenée de fluide. Dans l'exemple représenté à la figure 1, le conduit 10 est monté sur une bague 12 qui est engagée à coulissement sur le nez 14 de la pièce à main 1. Ce conduit 10 est raccordé à un flexible 16 par lequel arrive le fluide et sert à diriger un jet de fluide vers le champ opératoire. Néanmoins, l'extrémité 18 par laquelle le jet de fluide débouche du conduit 10 ne peut être disposée au-delà de la douille d'accouplement 4 car cela empêcherait le montage/démontage aisé de l'outil 6. Ainsi, lorsque l'outil 6 est long comme c'est le cas de la lame de scie représentée à la figure 1, le point de sortie du sérum physiologique du conduit 10 est éloigné de la zone de travail de l'outil. L'irrigation de la zone de travail est donc imprécise et ne se fait pas toujours en quantité suffisante.

Le document de brevet FR 2 635 676 A1 divulgue un dispositif d'accouplement entre un arbre moteur d'un instrument chirurgical et un outil selon le préambule de la revendication 1 annexée.

La présente invention a pour but de remédier à ce problème en procurant un dispositif d'accouplement d'un outil sur une pièce à main motorisée qui permet une irrigation précise et en quantité suffisante du champ opératoire.

A cet effet, la présente invention concerne un dispositif d'accouplement entre un arbre moteur d'un instrument chirurgical et une queue d'un outil selon la revendication 1 annexée à la présente demande de brevet.

Grâce à ces caractéristiques, la présente invention procure un dispositif pour l'accouplement d'un outil chirurgical sur l'arbre moteur d'un instrument chirurgical qui permet d'amener un fluide tel qu'un sérum physiologique au plus près de la zone de travail de l'outil. En effet, plutôt que d'amener le fluide au niveau de la zone de travail au moyen d'un conduit extérieur dont l'extrémité de déchargement du fluide ne peut s'étendre au-delà du point d'accouplement entre l'outil et l'arbre moteur de la pièce à main, la présente invention enseigne d'acheminer le fluide à l'intérieur de l'outil et de le faire émerger au plus près de la zone active dudit outil. De la sorte, on est assuré d'irriguer le champ opératoire de manière précise et en quantité suffisante pour garantir des conditions opératoires optimales, ce qui est très important en particulier pour les outils d'une certaine longueur.

Selon une caractéristique complémentaire de l'invention, le dispositif d'accouplement se compose d'une pièce support qui assure la liaison proprement dite entre la queue de l'outil et l'arbre motorisé de l'instrument chirurgical, un manchon sur lequel est couplée rigidement une douille étant disposé concentriquement autour de la pièce support pour définir une chambre d'amenée du sérum physiologique. La présente invention procure ainsi un dispositif d'accouplement qui se compose de trois pièces seulement et qui est donc à la fois simple à réaliser et extrêmement compact, ce dernier aspect facilitant grandement le maniement de l'instrument chirurgical par le praticien. En outre, l'ensemble des pièces qui compose le dispositif d'accouplement selon l'invention présente une symétrie générale de révolution, ce qui élimine les aspérités et autres angles vifs qui pourraient occasionner des blessures aux patients.

Selon une autre caractéristique de l'invention, le dispositif d'accouplement permet un verrouillage axial rigide entre la queue de l'outil et l'arbre moteur de l'instrument chirurgical grâce à un système de ressort de rappel et de bille de blocage. Quant au verrouillage en rotation, il est assuré par un plat prévu sur la queue de l'outil et sur lequel vient prendre appui un plat correspondant prévu sur le dispositif d'accouplement. De la sorte, l'outil est verrouillé aussi bien axialement que radialement pendant les phases de travail tout en pouvant être monté/démonté très facilement grâce au système à ressort et bille de blocage. Ce dernier aspect est très important, car il facilite considérablement la tâche du praticien qui est amené à utiliser plusieurs outils au cours d'une intervention.

D'autres caractéristiques et avantages de la présente invention ressortiront plus clairement de la description détaillée qui suit d'un mode de réalisation du dispositif d'accouplement selon l'invention, cet exemple étant donné à titre purement illustratif et non limitatif seulement en liaison avec le dessin annexé sur lequel :
- la figure 1, déjà citée, est une représentation d'un instrument chirurgical comprenant un système d'irrigation du champ opératoire selon l'art antérieur ;
- la figure 2 est une vue coupe longitudinale du dispositif d'accouplement selon l'invention en position de service ;
- la figure 3 est une vue en perspective de la pièce support ;
- la figure 4 est une vue analogue à celle de la figure 2, le dispositif d'accouplement selon l'invention étant représenté en position de libération de la queue de l'outil ;
- la figure 5 de dessus de l'outil sur laquelle est visible la surface plane qui permet le verrouillage radial de l'outil, et
- la figure 6 est une vue de dessous d'un second type d'outil comprenant un corps allongé et localement déformé en S qui s'étend entre la queue de l'outil et sa zone active et qui est traversé sur toute sa longueur par un canal dans lequel circule un fluide et qui débouche au niveau de la zone active.

La présente invention procède de l'idée générale inventive qui consiste à procurer un dispositif d'accouplement pour un instrument chirurgical motorisé qui permette notamment une irrigation précise et en quantité suffisante de la zone opératoire au moyen d'un fluide tel que du sérum physiologique. A cet effet, le dispositif d'accouplement permet à la fois de coupler rigidement l'outil à l'arbre moteur de l'instrument chirurgical et de mettre en communication un canal s'étendant axialement dans la queue de l'outil avec une source de fluide extérieure. De la sorte, le fluide émerge de la queue de l'outil au niveau de la zone active de celui-ci, c'est-à-dire au plus près du champ opératoire qui, masqué par des tissus, est autrement difficile d'accès notamment dans le cas d'outils d'une certaine longueur. D'autre part, le dispositif d'accouplement comprend un nombre limité de pièces, est compact et dépourvu d'aspérités, ce qui rend le maniement de l'instrument chirurgical plus aisé. Le praticien se fatigue moins et son geste est donc plus précis.

La présente invention va être décrite en liaison avec un instrument chirurgical motorisé plus particulièrement destiné à des opérations dans le domaine de la rhinoplastie. Il va néanmoins de soi que la présente invention n'est pas limitée à ce genre d'interventions et qu'elle s'applique de manière identique à tout type d'instrument chirurgical motorisé entraînant un outil dont la zone de travail a besoin d'être irriguée pour assurer les meilleures performances possibles en termes de coupe, d'abrasion, de perçage ou autre et l'élimination des déchets et du sang.

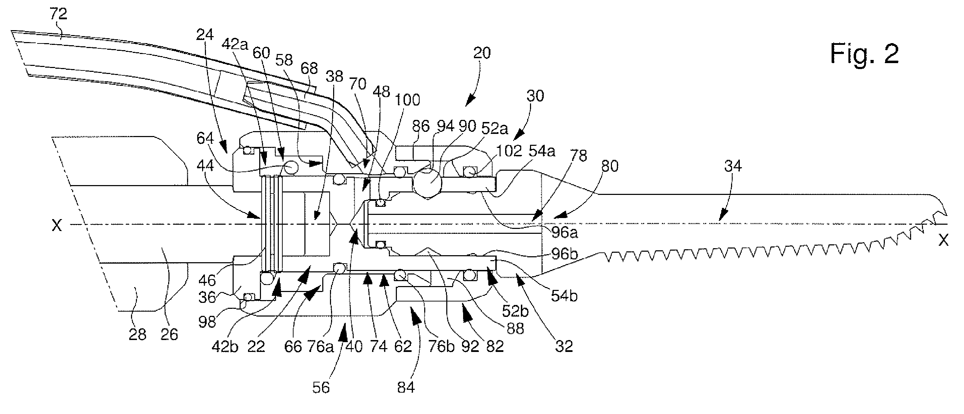

Désigné dans son ensemble par la référence numérique générale 20, le dispositif d'accouplement selon la présente invention est représenté en coupe longitudinale dans sa position de travail à la figure 2. Ce dispositif d'accouplement 20 comprend en premier lieu une pièce support 22 qui, à une extrémité arrière ou proximale 24, est portée par un arbre moteur 26 d'un instrument chirurgical 28 et qui, à une extrémité avant ou distale 30, porte une queue 32 d'un outil 34, en l'occurrence une scie. Comme on peut le voir à l'examen de la figure 2, la pièce support 22 est une pièce de forme générale cylindrique qui est terminée à son extrémité arrière 24 par une collerette circulaire ou embase 36 et dans laquelle sont usinés un premier et un second alésages respectivement 38 et 40. L'arbre moteur 26 de la pièce à main 28 est engagé dans le premier alésage 38 de la pièce support 22. De préférence, la pièce support 22 est chassée et goupillée sur l'arbre moteur 26. Néanmoins, selon une variante, la pièce support 22 pourra être faite d'une seule pièce avec l'arbre moteur 26. Dans le cas où elle est montée sur l'arbre moteur 26, la pièce support 22 est percée de deux trous 42a et 42b diamétralement opposés et l'arbre moteur 26 présente également un perçage 44. Ces deux trous 42a et 42b ainsi que le perçage 44 sont alignés pour le passage d'une goupille 46. La pièce support 22 est ainsi couplée rigidement à l'arbre moteur 26 de l'instrument chirurgical 28 tout en restant démontable pour des raisons de maintenance et de stérilisation. La queue 32 de l'outil 34 est engagée dans le second alésage 40 de la pièce support 22. L'outil 34 est couplé axialement et radialement avec la pièce support 22 d'une manière qui sera décrite en détail ci-dessous. La pièce support 22 est également percée d'un trou unique 48 qui forme conduit débouchant dans le second alésage 40 de même que d'au moins un, et préférentiellement trois trous traversants 50a, 50b et 50c régulièrement espacés le long du périmètre de cette pièce support 22 et qui débouchent eux aussi dans le second alésage 40. Enfin, la pièce support 22 présente à son extrémité avant 30 deux saillies 52a et 52b en arc de cercle diamétralement opposées dont les surfaces en regard 54a et 54b sont planes. Le rôle de ces différents éléments sera également décrit en détail ci-dessous.

En second lieu, le dispositif d'accouplement 20 selon l'invention comprend un manchon 56 disposé concentriquement autour de la pièce support 22. Ce manchon 56 présente une forme générale extérieure cylindrique et un espace intérieur également cylindrique présentant localement une réduction de diamètre 58. Cette réduction de diamètre 58 scinde ainsi le volume intérieur du manchon 56 en un premier logement 60 dont le diamètre est ajusté sur celui de l'embase 36 et en un second logement 62 dont le diamètre est ajusté sur le diamètre extérieur de la pièce support 22. Un ressort de rappel 64 est disposé à l'intérieur du premier logement 60, en appui à une extrémité arrière contre l'embase 36 et à une extrémité avant contre le fond 66 dudit premier logement 60. Un embout coudé 68 en acier inoxydable est inséré dans un premier conduit 70 réalisé préférentiellement en oblique par rapport à l'axe général de symétrie longitudinale X-X du dispositif d'accouplement 20. Un tuyau flexible 72 d'amenée d'un fluide tel que du liquide physiologique est raccordé à l'embout coudé 68.

Le conduit 70 débouche dans une chambre annulaire 74 d'amenée d'un fluide définie par l'intervalle laissé libre entre la paroi du second logement 62 du manchon 56 et la surface extérieure de la pièce support 22. L'étanchéité de la chambre annulaire 74 est assurée au moyen de deux joints 76a et 76b placés sensiblement à l'extrémité arrière et à l'extrémité avant du second logement 62. La chambre annulaire 74 communique avec le conduit 48 mentionné ci-dessus qui débouche dans le second alésage 40 de la pièce support 22. Un joint d'étanchéité 100 doit être monté sur l'extrémité arrière de la queue 32 de l'outil 34 pour éviter que le liquide physiologique ne fuie vers l'extérieur. Ainsi, le liquide physiologique amené depuis l'extérieur via le tuyau flexible 72 et l'embout coudé 68 va successivement s'écouler dans le premier conduit 70, puis la chambre annulaire 74 et le second conduit 48 avant de venir remplir le fond de l'alésage 40. Un canal 78 est usiné dans la queue 32 de l'outil 34 et débouche en un endroit de la longueur de la queue 32, de préférence au niveau de la zone active 80 de l'outil 34, c'est-à-dire, dans le cas où l'outil 34 est une lame de scie, au niveau des dents de cette scie. Ainsi, le fluide physiologique qui a envahit le fond de l'alésage 40 va s'échapper en s'écoulant à travers le canal 78 et émerger au niveau de la zone active 80 de l'outil 34. En forçant le liquide physiologique à emprunter un chemin qui le conduit depuis l'extérieur du dispositif d'accouplement 20 jusqu'au centre de la queue 32 de l'outil 34, on permet de déplacer le point où émerge le liquide physiologique au plus près de la partie active de l'outil. L'irrigation du champ opératoire se fait donc de manière précise et en quantité suffisante pour garantir des conditions opératoires optimales.

On s'intéresse maintenant au verrouillage/déverrouillage de l'outil sur l'arbre moteur de l'instrument chirurgical. A cet effet, le dispositif d'accouplement 20 selon l'invention comprend, en troisième et dernier lieu, une douille 82 disposée concentriquement autour de la pièce support 22 et couplée rigidement au manchon 56 de préférence par chassage ou autre technique de fixation. Pour le montage de la douille 82 sur le manchon 56, ce dernier présente, du côté de son extrémité avant 84, un rebord annulaire 86 dont le diamètre extérieur est adapté au diamètre intérieur du logement 88 défini par la douille 82. Comme on peut le voir à l'examen de la figure 2, l'un des trois trous traversants 50a pratiqué dans la pièce support 22 et qui débouche dans le second alésage 40 sert de siège à une bille de blocage 90. La bille de blocage 90 est retenue dans le siège 50a qui présente à cet effet dans son fond un diamètre légèrement inférieur au plus grand diamètre de la bille 90.

La bille de blocage 90 fait saillie de part et d'autre de la pièce support 22 d'un côté dans l'alésage 40 et de l'autre côté dans le logement 88 défini par la douille 82. Plus précisément, en position de service du dispositif d'accouplement 20 telle qu'illustrée à la figure 2, la bille de blocage 90 fait saillie, du côté du second alésage 40 dans une gorge 92 à profil en V pratiquée dans le pourtour extérieur de la queue 32 de l'outil 34 et est maintenue dans cette gorge 92 par le manchon 56 qui est forcé en direction de la bille 90 par la force de rappel élastique du ressort 64. On voit que le manchon 56 est en appui contre la bille de blocage 90 via une surface tronconique 94 que présente le rebord annulaire 86 sur son périmètre intérieur. La bille de blocage 90 assure ainsi le couplage axial entre l'outil 34 et l'arbre moteur 26 de l'instrument chirurgical 28 via la pièce support 22. Quand au blocage en rotation de l'outil 34 sur l'arbre moteur 26, il est assuré par la pièce support 22 proprement dite dont les saillies 52a et 52b viennent, par leurs surfaces planes 54a et 54b en regard, en appui sur des surfaces planes correspondantes 96a et 96b ménagées sur la queue 32 de l'outil. En position de travail, l'outil 34 est donc couplé rigidement à l'arbre moteur 26.

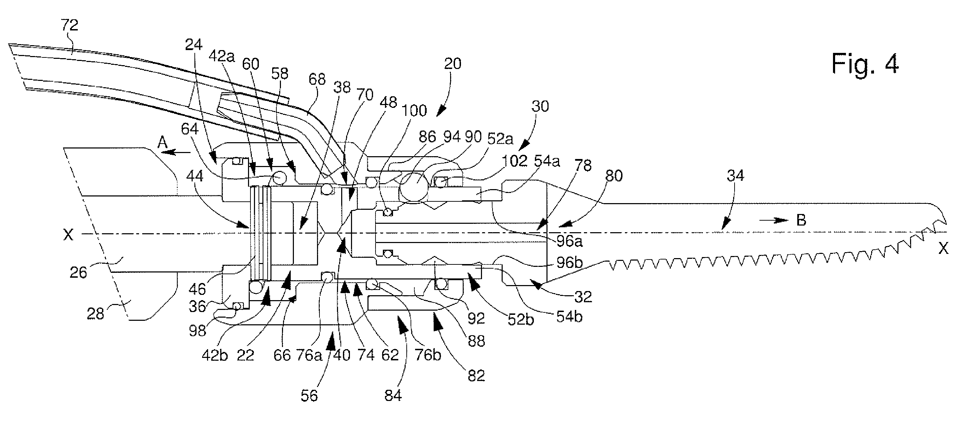

On examine maintenant en liaison avec la figure 4 l'opération de démontage de l'outil 34. A cette fin, l'utilisateur exerce une traction vers l'arrière à l'encontre de la force de rappel du ressort 64 sur l'ensemble formé par le manchon 56 et la douille 82 (voir flèche A). Simultanément, l'utilisateur exerce une traction vers l'avant sur l'outil 34 (voir flèche B). Sous l'effet de cette traction, la bille de blocage 90 qui n'est plus maintenue en place dans la gorge 92 par la surface tronconique 94, roule sur le périmètre extérieur de la queue 32 de l'outil 34 et remonte dans le logement 88 défini par la douille 82 qui se trouve juste au dessus de la bille 90. A ce moment là, plus rien ne s'oppose à l'extraction de la queue 32 de l'outil 34. Quant à la bille de blocage 90, une fois la queue 32 de l'outil 34 extraite de l'alésage 40, elle retombe dans son siège défini par le trou 50a.

A l'extrémité arrière du dispositif d'accouplement 20, un joint d'étanchéité 98 peut être prévu entre l'embase 36 et le premier logement 60. Un joint d'étanchéité 102 peut également être prévu entre la douille 82 et la pièce support 22 pour éviter que les résidus de l'opération ne pénètrent dans le dispositif d'accouplement 20.

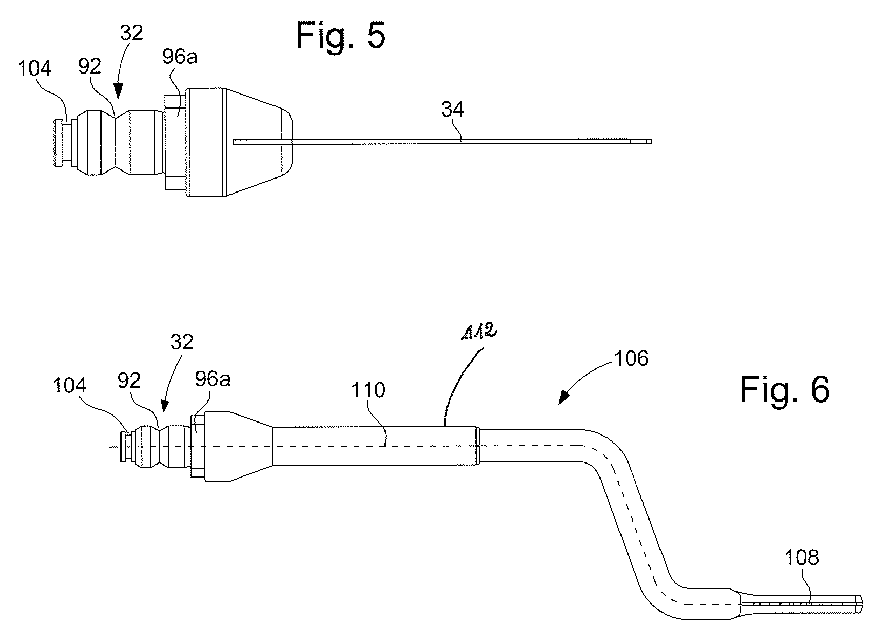

La figure 5 est une vue de dessus de l'outil 34 sur laquelle on voit notamment la surface plane 96a sur laquelle vient prendre appui par sa surface plane correspondante 54a la saillie 52a prévue à l'extrémité avant de la pièce support 22 pour le blocage en rotation de l'outil 34. La figure 5 révèle également une rainure circulaire 104 dans laquelle est logé le joint d'étanchéité 100. On voit également la gorge en V 92 dans laquelle vient faire saillie la bille 90 pour le blocage axial de l'outil 34.

La figure 6 est une vue de dessous d'un autre type d'outil. Désigné dans son ensemble par la référence numérique générale 106, cet outil se distingue de celui représenté aux figures 2, 4 et 5 uniquement en ce que sa queue 32 qui sert à son montage sur l'arbre moteur de l'instrument chirurgical via la pièce support 22 selon l'invention est reliée à sa partie active 108, en l'occurrence une lame de scie en demi-cercle, via un corps allongé 112. Un canal 110 (représenté en pointillés sur la figure 6) s'étend à travers l'outil 106, ce canal 110 étant usiné dans la queue 32 et dans le corps 112 de l'outil 106 et débouchant en un endroit de la longueur dudit corps de l'outil 106, de préférence au voisinage de la zone active 108 de celui-ci. Après usinage du canal 110 dans la queue 32 et le corps 112 de l'outil 106, on peut impartir à l'outil 106 la forme désirée, par exemple en le conformant partiellement en S comme représenté au dessin. On comprend ainsi qu'on peut faire déboucher le liquide physiologique au plus près de la zone active de l'outil, même lorsque celui-ci est de longueur conséquente.

## Revendications

1. Dispositif d'accouplement entre un arbre moteur (26) d'un instrument chirurgical (28) et un outil (34; 106), dans lequel le dispositif d'accouplement (20) comprend un élément d'amenée d'un fluide alimenté depuis l'extérieur du dispositif d'accouplement (20) et communiquant avec un canal (78 ; 110) ménagé dans l'outil (34 ; 106) et qui débouche en un endroit de la longueur de l'outil (34 ; 106), le dispositif d'accouplement (20) comprenant également un manchon (56) comportant un premier conduit (70) par lequel le fluide arrive de l'extérieur du dispositif d'accouplement (20), **caractérisé en ce qu'**il comprend une pièce support (22) qui, à une extrémité arrière (24), est couplée rigidement à l'arbre moteur (26) de l'instrument chirurgical (28) et qui, à une extrémité avant (30), porte la queue (32) de l'outil (34; 106), et **en ce que** le manchon (56) est disposé concentriquement autour de la pièce support (22) et délimite avec cette pièce support (22) une chambre annulaire (74) d'amenée du fluide.

2. Dispositif d'accouplement selon la revendication 1, **caractérisé en ce que** la pièce support (22) est faite d'une pièce avec l'arbre moteur (26) de l'instrument chirurgical (28).

3. Dispositif d'accouplement selon la revendication 1, **caractérisé en ce que** la pièce support (22) est chassée et goupillée sur l'arbre moteur (26) de l'instrument chirurgical (28).

4. Dispositif d'accouplement selon l'une quelconque des revendications 1 à 3, **caractérisé en ce que**, à son extrémité avant (30), la pièce support (22) présente un alésage (40) en communication avec l'élément d'amenée du fluide et dans lequel est engagée la queue (32) de l'outil (34 ; 106).

5. Dispositif d'accouplement selon l'une quelconque des revendications 1 à 4, **caractérisé en ce que** le premier conduit (70) communique via la chambre d'amenée de fluide (74) avec un second conduit (48) ménagé dans la pièce support (22) et qui lui-même communique avec le canal (78 ; 110) ménagé dans la queue (32) de l'outil (34 ; 106).

6. Dispositif d'accouplement selon la revendication 5, **caractérisé en ce que** la chambre d'amenée du fluide (74) est rendue étanche à ses première et seconde extrémités par deux joints d'étanchéité (76a, 76b) et **en ce qu'**un joint d'étanchéité (100) est monté sur l'extrémité arrière de la queue (32) de l'outil (34).

7. Dispositif d'accouplement selon l'une quelconque des revendications 1 à 6, **caractérisé en ce qu'**il comprend également une douille (82) disposée concentriquement autour de la pièce support (22) et couplée rigidement au manchon (56), l'ensemble formé par le manchon (56) et la douille (82) assurant le blocage axial de la queue (32) de l'outil (34 ; 106) sur la pièce support (22).

8. Dispositif d'accouplement selon la revendication 7, **caractérisé en ce que** l'ensemble formé par le manchon (56) et la douille (82) est rappelé axialement en position de blocage de l'outil (34 ; 106) sur la pièce support (22) par un ressort (64), une bille de blocage (90) logée dans un siège (50a) pratiqué dans la pièce support (22) étant forcée par le manchon (56) dans une gorge (92) ménagée sur le pourtour de la queue (32) de l'outil (34 ; 106).

9. Dispositif d'accouplement selon la revendication 8, **caractérisé en ce que**, lorsque l'ensemble formé par le manchon (56) et la douille (82) est amené en positon de libération de la queue (32) de l'outil (34 ; 106) à l'encontre de la force de rappel du ressort (64), la bille de blocage (90) se retrouve au niveau d'un logement intérieur (88) délimité par la douille (82), le diamètre de ce logement (88) étant suffisant pour permettre à la bille de blocage (90) de sortir de la gorge (92) ménagée sur le pourtour de la queue (32) de l'outil (34 ; 106) lorsque la queue (32) de l'outil (34 ; 106) est retirée de la pièce support (22).

10. Dispositif d'accouplement selon l'une quelconque des revendications 8 ou 9, **caractérisé en ce que** le ressort de rappel (64) est en appui à une première extrémité contre une embase (36) prévue sur la pièce support (22) et contre un épaulement intérieur (58) prévu dans le manchon (56) à une seconde extrémité.

11. Dispositif d'accouplement selon l'une quelconque des revendications 1 à 10, **caractérisé en ce que**, pour son immobilisation radiale, la queue (32) de l'outil (34 ; 106) présente une surface plane (96a, 96b) sur laquelle vient prendre appui une surface plane correspondante (54a, 54b) prévue à la seconde extrémité de la pièce support (22).

12. Système chirurgical comprenant un outil (34; 106) et un dispositif d'accouplement (20) selon l'une quelconque des revendications 1 à 11 entre un arbre moteur (26) d'un instrument chirurgical (28) et l'outil (34 ; 106), l'outil (34 ; 106) comprenant une queue (32) par laquelle il est monté sur l'arbre moteur (26) de l'instrument chirurgical (28) via le dispositif d'accouplement (20), la queue (32) étant reliée directement ou par le biais d'un corps (112) à une zone active (80 ; 108) de l'outil (34 ; 106), un canal (78 ; 110) passant à travers la queue (32) et, le cas échéant, à travers le corps (112) de l'outil (34 ; 106) débouchant en un endroit de la longueur de l'outil (34 ; 106), **caractérisé en ce que**, pour son immobilisation radiale, la queue (32) de l'outil (34 ; 106) présente une surface plane (96a, 96b) sur laquelle vient prendre appui une surface plane correspondante (54a, 54b) d'une pièce support (22) qui, à une extrémité arrière (24), est couplée rigidement à l'arbre moteur (26) de l'instrument chirurgical (28) et qui, à une extrémité avant (30), porte la queue (32) de l'outil (34 ; 106), et **en ce que**, pour son immobilisation axiale, la queue (32) de l'outil (34 ; 106) présente une gorge (92) ménagée sur le pourtour de la queue (32) de l'outil (34 ; 106) et dans laquelle fait saillie une bille de blocage (90) logée dans un siège (50a) pratiqué dans la pièce support (22).

## Patentansprüche

1. Kopplungsvorrichtung zwischen einer Motorwelle (26) eines chirurgischen Instruments (28) und einem Werkzeug (34; 106), wobei die Kopplungsvorrichtung (20) ein Fluidzufuhrelement umfasst, das von außerhalb der Kopplungsvorrichtung (20) gespeist wird und mit einem in dem Werkzeug (34; 106) ausgebildeten Kanal (78; 110) kommuniziert, der an einem Ort auf der Länge des Werkzeugs (34; 106) mündet, wobei die Kopplungsvorrichtung (20) außerdem eine Muffe (56) umfasst, die eine erste Leitung (70) aufweist, durch die das Fluid von außerhalb der Kopplungsvorrichtung (20) ankommt, **dadurch gekennzeichnet, dass** sie ein Tragteil (22) umfasst, das an einem hinteren Ende (24) starr mit der Motorwelle (26) des chirurgischen Instruments (28) gekoppelt ist und das an einem vorderen Ende (30) den Schaft (32) des Werkzeugs (34; 106) trägt, und dass die Muffe (56) konzentrisch um das Tragteil (22) angeordnet ist und mit diesem Tragteil (22) eine Fluidzufuhr-Ringkammer (74) begrenzt.

2. Kopplungsvorrichtung nach Anspruch 1, **dadurch gekennzeichnet, dass** das Tragteil (22) mit der Motorwelle (26) des chirurgischen Instruments (28) einteilig ausgebildet ist.

3. Kopplungsvorrichtung nach Anspruch 1, **dadurch gekennzeichnet, dass** das Tragteil (22) auf die Motorwelle (26) des chirurgischen Instruments (28) getrieben ist und damit verstiftet ist.

4. Kopplungsvorrichtung nach einem der Ansprüche 1 bis 3, **dadurch gekennzeichnet, dass** das Tragteil (22) an seinem vorderen Ende (30) eine Bohrung (40) aufweist, die mit dem Fluidzufuhrelement kommuniziert und mit der der hintere Teil (32) des Werkzeugs (34; 106) in Eingriff ist.

5. Kopplungsvorrichtung nach einem der Ansprüche 1 bis 4, **dadurch gekennzeichnet, dass** die erste Leitung (70) über die Fluidzufuhrkammer (74) mit einer zweiten Leitung (48) kommuniziert, die in dem Tragteil (22) ausgebildet ist und die ihrerseits mit dem Kanal (78; 110) kommuniziert, der in dem Schaft (32) des Werkzeugs (34; 106) ausgebildet ist.

6. Kopplungsvorrichtung nach Anspruch 5, **dadurch gekennzeichnet, dass** die Fluidzufuhrkammer (74) an ihrem ersten und an ihrem zweiten Ende durch zwei Dichtungen (76a, 76b) abgedichtet ist und dass an dem hinteren Ende des Schafts (32) des Werkzeugs (34) eine Dichtung (100) montiert ist.

7. Kopplungsvorrichtung nach einem der Ansprüche 1 bis 6, **dadurch gekennzeichnet, dass** sie außerdem eine Hülse (82) umfasst, die konzentrisch um das Tragteil (22) angeordnet ist und mit der Muffe (56) starr gekoppelt ist, wobei die durch die Muffe (56) und die Hülse (82) gebildete Anordnung die axiale Blockierung des Schafts (32) des Werkzeugs (34; 106) am Tragteil (22) sicherstellt.

8. Kopplungsvorrichtung nach Anspruch 7, **dadurch gekennzeichnet, dass** die durch die Muffe (56) und die Hülse (82) gebildete Anordnung durch eine Feder (64) in die Blockierposition des Werkzeugs (34; 106) an dem Tragteil (22) axial zurückgestellt wird, wobei eine Blockierkugel (90), die sich in einem in dem Tragteil (22) ausgebildeten Sitz (50a) befindet, durch die Muffe (56) in eine Kehle (92) gedrängt wird, die am Umfang des Schafts (32) des Werkzeugs (34; 106) ausgebildet ist.

9. Kopplungsvorrichtung nach Anspruch 8, **dadurch gekennzeichnet, dass** dann, wenn die durch die Muffe (56) und die Hülse (82) gebildete Anordnung entgegen der Rückstellkraft der Feder (64) in die Position zur Freigabe des Schafts (32) des Werkzeugs (34; 106) geführt ist, die Blockierkugel (90) sich auf Höhe eines inneren Aufnahmesitzes (88) befindet, das durch die Hülse (82) begrenzt ist, wobei der Durchmesser dieses Aufnahmesitzes (88) ausreicht, um der Blockierkugel (90) zu ermöglichen, die am Umfang des Schafts (32) des Werkzeugs (34; 106) ausgebildete Kehle (92) zu verlassen, wenn der hintere Teil (32) des Werkzeugs (34; 106) aus dem Tragteil (22) herausgezogen wird.

10. Kopplungsvorrichtung nach einem der Ansprüche 8 oder 9, **dadurch gekennzeichnet, dass** sich die Rückstellfeder (64) an einem ersten Ende an einem Sockel (36) abstützt, der an dem Tragteil (22) vorgesehen ist, und sich an einem zweiten Ende an einer inneren Schulter (58) abstützt, die in der Muffe (56) vorgesehen ist.

11. Kopplungsvorrichtung nach einem der Ansprüche 1 bis 10, **dadurch gekennzeichnet, dass** der hintere Teil (32) des Werkzeugs (34; 106) eine ebene Oberfläche (96a, 96b) aufweist, auf der sich eine entsprechende ebene Oberfläche (54a, 54b) abstützt, die am zweiten Ende des Tragteils (22) vorgesehen ist, um den Schaft (32) des Werkzeugs (34; 106) unbeweglich zu machen.

12. Chirurgisches System, das ein Werkzeug (34; 106) und eine Kopplungsvorrichtung (20) nach einem der Ansprüche 1 bis 11 zwischen einer Motorwelle (26) eines chirurgischen Instruments (28) und dem Werkzeug (34; 106) umfasst, wobei das Werkzeug (34; 106) einen Schaft (32) aufweist, durch den es über die Kopplungsvorrichtung (20) an der Motorwelle (26) des chirurgischen Instruments (28) montiert ist, wobei der hintere Teil (32) direkt oder durch einen Körper (112) mit einer aktiven Zone (80; 108) des Werkzeugs (34; 106) verbunden ist, wobei ein Kanal (78; 110) durch den Schaft (32) und gegebenenfalls durch den Körper (112) des Werkzeugs (34; 106) verläuft und an einem Ort auf der Länge des Werkzeugs (34; 106) mündet, **dadurch gekennzeichnet, dass** der hintere Teil (32) des Werkzeugs (34; 106), um ihn radial unbeweglich zu machen, eine ebene Oberfläche (96a, 96b) aufweist, auf der sich eine entsprechende ebene Oberfläche (54a, 54b) eines Tragteils (22) abstützt, das an seinem hinteren Ende (24) starr mit der Motorwelle (26) des chirurgischen Instruments (28) gekoppelt ist und an seinem vorderen Ende (30) den Schaft (32) des Werkzeugs (34; 106) trägt, und dass der hintere Teil (32) des Werkzeugs (34; 106), um ihn axial unbeweglich zu machen, eine Kehle (92), die am Umfang des Schafts (32) des Werkzeugs (34; 106) ausgebildet ist, aufweist, in die eine Blockierkugel (90) vorsteht, die in einem Sitz (50a) aufgenommen ist, der in dem Tragteil (22) ausgebildet ist.

## Claims

1. Coupling device between a drive shaft (26) of a surgical instrument (28) and a tool (34; 106), wherein the coupling device (20) includes a fluid feed element fed from outside the coupling device (20) and communicating with a channel (78; 110) arranged in the tool (34; 106) and which emerges at a location along the length of the tool (34; 106), the coupling device (20) also comprising a sleeve (56) including a first pipe (70) through which the fluid arrives from outside the coupling device (20), **characterized in that** it includes a support part (22) which, at a rear end (24), is rigidly coupled to the drive shaft (26) of the surgical instrument (28) and which, at a front end (30), carries the shank (32) of the tool (34; 106), and **in that** sleeve (56) is arranged concentrically around the support part (22) and delimits, with said support part (22), an annular fluid inlet chamber (74).

2. Coupling device according to claim 1, **characterized in that** the support part (22) is integral with the drive shaft (26) of the surgical instrument (28).

3. Coupling device according to claim 1, **characterized in that** the support part (22) is driven and pinned onto the drive shaft (26) of the surgical instrument (28).

4. Coupling device according to any of claims 1 to 3, **characterized in that**, at the front end thereof (30), the support part (22) has a bore (40) that communicates with the fluid feed element and in which the shank (32) of the tool (34; 106) is engaged.

5. Coupling device according to any of claims 1 to 4, **characterized in that** the first pipe (70) communicates via the fluid inlet chamber (74) with a second pipe (48) arranged in the support part (22) and which itself communicates with the channel (78; 110) arranged in the shank (32) of the tool (34; 106).

6. Coupling device according to claim 5, **characterized in that** the fluid inlet chamber (74) is sealed at the first and second ends thereof by two sealing gaskets (76a, 76b) and **in that** a sealing gasket (100) is mounted on the rear end of the tool (34) shank (32).

7. Coupling device according to any of claims 1 to 6, **characterized in that** it also includes a bushing (82) arranged concentrically around the support part (22) and rigidly coupled to the sleeve (56), the assembly formed by the sleeve (56) and the bushing (82) locking the tool (34; 106) shank (32) axially on the support part (22).

8. Coupling device according to claim 7, **characterized in that** the assembly formed by the sleeve (56) and the bushing (82) is returned axially to the tool (34; 106) locking position on the support part (22) by a spring (64), a lock ball (90) housed in a seat (50a) made in the support part (22) being forced by the sleeve (56) into a groove (92) arranged on the periphery of the shank (32) of the tool (34; 106).

9. Coupling device according to claim 8, **characterized in that**, when the assembly formed by the sleeve (56) and the bushing (82) is brought into the position for releasing the tool (34; 106) shank (32) against the return force of the spring (64), the lock ball (90) is located by an inner housing (88), delimited by the bushing (82), the diameter of said housing (88) being sufficient to enable the lock ball (90) to exit the groove (92) arranged on the periphery of the shank (32) of the tool (34; 106) when the tool (34; 106) shank (32) is removed from the support part (22).

10. Coupling device according to any of claims 8 or 9, **characterized in that** the return spring (64) abuts, at a first end, against a base (36) provided on the support part (22) and against an inner shoulder (58) provided in the sleeve (56) at a second end.

11. Coupling device according to any of claims 1 to 10, **characterized in that**, for radial immobilisation, the tool (34, 106) shank (32) has a flat surface (96a; 96b) on which abuts a corresponding flat surface (54a, 54b) at the second end of the support part (22).

12. Chirurgical system comprising a tool (34; 106) and a coupling device (20) according to any of claims 1 to 11 between a drive shaft (26) of a surgical instrument (28) and the tool (34; 106), the tool (34; 106) comprising a shank (32) via which it is mounted on the drive shaft (26) of the surgical instrument (28) via the coupling device (20), said shank (32) being connected directly, or via a body (112), to an active area (80; 108) of said tool (34; 106), a channel (78; 110) passing through the shank (32) and, where appropriate, through the body (112) of the tool (34; 106) emerging at a location along the length of the tool (34; 106), **characterized in that**, for radial immobilisation, the tool (34; 106) shank (32) has a flat surface (96a; 96b) on which abuts a corresponding flat surface (54a, 54b) of a support part (22) which, at a rear end (24), is rigidly coupled to the drive shaft (26) of the surgical instrument (28) and which, at a front end (30), carries the shank (32) of the tool (34; 106), and **in that**, for axial immobilisation, the tool (34; 106) shank (32) has a groove (92) arranged on the periphery of the tool (34; 106) shank (32) and in which a lock ball (90) housed in a seat (50a) made in the support part (22) projects.
